(19) Europäisches Patentamt
European Patent Office
Office européen des brevets

(11) **EP 1 710 573 A1**

(12) **DEMANDE DE BREVET EUROPEEN**

(43) Date de publication:
**11.10.2006 Bulletin 2006/41**

(51) Int Cl.:
*G01N 31/22* (2006.01)    *G01N 33/00* (2006.01)

(21) Numéro de dépôt: **06370011.6**

(22) Date de dépôt: **06.04.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **06.04.2005 FR 0503428**

(71) Demandeur: **UNIVERSITE DES SCIENCES ET TECHNOLOGIES DE LILLE**
**59655 Villeneuve d'Ascq (FR)**

(72) Inventeurs:
• **Déchaux, Jean-Claude,**
  **c/o Universite des Sciences**
  **59655 Villeneuve d'Ascq (FR)**
• **Triki, Nicolas,**
  **c/o Universite des Sciences**
  **59655 Villeneuve d'Ascq (FR)**

(74) Mandataire: **Duthoit, Michel Georges André**
**Bureau Duthoit Legros Associés**
**Conseil en Propiété Industrielle**
**96/98, Boulevard Carnot,**
**B.P. 105**
**59027 Lille Cedex (FR)**

(54) **Procédé et dispositif de mesure d'un taux de monoxyde de carbone dans un fluide**

(57)    L'invention concerne un procédé de mesure du taux volumique de monoxyde de carbone dans un fluide gazeux donné.

Selon l'invention, on fait passer un volume donné dudit fluide dans un temps donné, à travers un élément réactif pulvérulent (2) comprenant un réactif sélectif adsorbé sur un gel de silice, et ayant une réaction colorée avec ledit monoxyde de carbone présent dans ledit fluide.

L'invention concerne également un procédé de préparation d'un réactif conçu pour la mise en oeuvre du procédé de mesure du taux volumique de monoxyde de carbone. La préparation d'un tel réactif comprend les étapes suivantes :
- une étape de mélange de chlorure de palladium avec du potassium disulfate dans de l'eau distillée sous agitation,
- une étape de filtration de la suspension obtenue,
- une étape de lavage du précipité obtenu, avec un solvant partiellement organique,
- une étape de séchage du précipité.

L'invention concerne en outre un dispositif conçu pour la mise en oeuvre d'un procédé de mesure du taux volumique de monoxyde de carbone dans un fluide donné, et particulièrement dans les gaz d'échappement d'un système à combustion.

FIG.2

EP 1 710 573 A1

**Description**

L'invention concerne un procédé de mesure du taux volumique de monoxyde de carbone dans un fluide gazeux donné, ainsi qu'un dispositif conçu pour la mise en oeuvre du procédé.

Plus particulièrement, l'application principale de l'invention est la détermination du taux volumique de monoxyde de carbone dans les gaz d'échappement d'un système à combustion.

Actuellement, on connaît différents dispositifs et procédés de mesure du taux d'un gaz dans un fluide, tel que la teneur en alcool éthylique dans l'haleine.

Ces dispositifs fonctionnent sur la base d'une réaction chimique comprenant un tube de réactif sélectif se colorant en fonction de la quantité de gaz qui le traverse. Toutefois, ces dispositifs sont peu fiables et ne sont pas adaptés à la mesure du taux d'oxyde de carbone, dans un fluide gazeux donné.

On connaît aussi des appareils de mesure très sophistiqués mettant en oeuvre un spectromètre. Ces dispositifs permettent la mesure du taux de monoxyde de carbone, mais sont assez complexes et restent relativement onéreux.

Le but de la présente invention est de proposer un procédé de mesure du taux en volume de monoxyde de carbone dans un fluide gazeux donné qui pallie les inconvénients précités, notamment au niveau de la fiabilité des résultats.

Un autre but de l'invention est de proposer un dispositif conçu pour la mise en oeuvre dudit procédé qui permet d'aboutir à un dispositif moins cher et moins complexe.

D'autres buts et avantages de l'invention apparaîtront au cours de la description qui va suivre qui n'est donnée qu'à titre indicatif et qui n'a pas pour but de la limiter.

Selon la présente invention, le procédé de mesure du taux volumique de monoxyde de carbone dans un fluide gazeux donné est caractérisé par le fait qu'on fait passer un volume donné dudit fluide dans un temps donné, à travers un élément réactif pulvérulent, comprenant un réactif sélectif adsorbé sur un gel de silice, et ayant une réaction colorée avec ledit monoxyde de carbone présent dans ledit fluide.

L'invention concerne également un procédé de préparation du réactif conçu pour la mise en oeuvre du procédé de mesure du taux volumique de monoxyde de carbone dans un fluide gazeux donné, et ayant une réaction colorée avec ledit monoxyde de carbone. Ce procédé comprend les étapes suivantes :

- une étape de mélange du chlorure de palladium avec du potassium disulfate dans de l'eau distillée sous agitation,
- une étape de filtration de la suspension obtenue,
- une étape de lavage du précipité obtenu,
- une étape de séchage du précipité.

L'invention concerne également un dispositif pour mesurer le taux volumique de monoxyde de carbone dans un fluide donné, conçu pour la mise en oeuvre du procédé tel que mentionné plus haut, caractérisé par le fait qu'il comprend un élément réactif pulvérulent comprenant ledit réactif sélectif, adsorbé sur un gel de silice, et ayant une réaction colorée avec ledit monoxyde de carbone présent dans ledit fluide, et des moyens pour contrôler le passage d'un volume donné, dans un temps donné, dudit fluide.

L'invention concerne en outre un procédé de mesure, tel que décrit plus haut, de mise en oeuvre dans un dispositif utilisé dans le procédé de mesure du taux volumique de monoxyde de carbone tel que décrit ci-dessus. Ce procédé consiste à :

- rompre l'étanchéité du système afin de laisser pénétrer ledit fluide gazeux donné dans le dispositif,
- provoquer un transfert de la phase gazeuse à l'intérieur dudit dispositif afin que ledit fluide gazeux passe dans ledit dispositif selon un volume donné et un temps donné.

L'invention concerne en outre l'utilisation d'un tel dispositif dans la détermination du taux volumique de monoxyde de carbone dans les gaz d'échappement dans un système à combustion.

L'invention sera mieux comprise à la lecture de la description suivante, accompagnée des figures en annexe parmi lesquelles :

- la figure 1 représente schématiquement un dispositif pour mesurer le taux d'émission de monoxyde de carbone selon l'invention en état de repos,
- la figure 2 représente schématiquement un dispositif pour mesurer le taux d'émission de monoxyde de carbone selon l'invention sous dépression.

L'invention concerne tout d'abord un procédé de mesure du taux volumique de monoxyde de carbone dans un fluide donné.

Selon l'invention, on fait passer un volume donné dudit fluide dans un temps donné à travers un élément réactif pulvérulent comprenant un réactif sélectif adsorbé sur un gel de silice, et ayant une réaction colorée avec ledit monoxyde de carbone présent dans ledit fluide, dont la synthèse est décrite plus loin.

Selon un mode particulier de la présente invention, le réactif sélectif ayant une réaction colorée avec ledit monoxyde de carbone est le complexe di-potassium pallado bisulfite.

Le complexe di-potassium pallado bisulfite, lors du passage de monoxyde de carbone, réagit avec celui-ci en changeant de coloration.

La coloration est due à la formation de palladium métallique qui donne une couleur brun foncé, selon la réaction suivante :

$$K_2Pd(SO_3)_2 + CO \rightarrow CO_2 + SO_2 + Pd + K_2SO_3$$

Afin d'obtenir de bons résultats, et selon la présente invention, le volume dudit fluide aspiré se situe entre 5 et 35 ml et le temps de passage à travers l'élément réactif est de 2 min à 10 min.

On a obtenu de bons résultats avec un volume aspiré de 20 ml et un temps de passage de 2 min.

Les résultats de l'étude de la réaction entre le complexe di-potassium pallado bisulfite et le monoxyde de carbone ont montré l'influence de divers paramètres sur les colorations obtenues sur le tube, entre autres, la teneur en di-potassium pallado bisulfite dans la solution réactive, et la vitesse d'injection.

Quelques résultats sont présentés dans le tableau ci-dessous donné à titre d'exemple non exhaustif.

TABLEAU I

| N° Tube | Masse de complexe en g | % CO injecté | Temps d'injection mn | RESULTAT VISUEL |
|---------|------------------------|--------------|----------------------|-----------------|
| 1 | 0,1 | 5 | 10 | Coloration brun foncé totale après injection de 5 ml |
| 2 | 0,1 | 0,5 | 10 | Coloration brun foncé totale après injection de 12 ml |
| 3 | 0,2 | 0,5 | 10 | Coloration brun foncé immédiate avec front d'avancement très net |

Dans le cas du tube 1, pour préparer la phase réactive pulvérulente, on a dissout 0,1 mg de complexe di-potassium pallado bisulfite dans 50 ml d'eau et ensuite la solution a été mise en contact durant un temps optimal avec 2 g de gel de silice. Après séchage, le gel est alors placé dans un tube où l'on introduit 20 ml d'un mélange contenant 0,5 % de CO, ceci pendant 10 min. La dernière colonne décrit le résultat visuel obtenu.

Pour les cas des tubes 2 et 3, on a suivi le même procédé dans des conditions expérimentales indiquées dans le tableau.

Le premier constat que l'on peut faire, au vu de ce tableau de résultats, est la nette influence de la concentration en complexe mise en solution aqueuse pour réaliser l'adsorption sur le gel de silice. D'une part, la teinte de la coloration observée (brun foncé sur un solide pulvérulent initialement jaune) est plus intense, et d'autre part, cette concentration supérieure permet de distinguer un front d'avancement très net, condition indispensable à une bonne lecture à l'oeil pour que le test soit fiable. En effet, la vitesse de transfert du fluide gazeux prélevé doit obligatoirement être voisine de la vitesse de la réaction chimique utilisée, faute de quoi le CO passera sur le complexe réactif adsorbé sans avoir le temps de réagir avec lui.

Enfin, on constate que la coloration progresse plus rapidement dans le tube lorsque la vitesse d'injection augmente.

Selon un autre mode de réalisation de la présente invention, le volume de phase réactive présente est de l'ordre de 1 à 3 g. Elle est constituée surtout de gel de silice, car la masse de $K_2Pd(SO_3)_2$ qui y est adsorbée est nettement inférieure.

La présente invention concerne également un procédé de préparation dudit réactif adsorbé, conçu pour la mise en oeuvre du procédé de mesure du taux volumique de monoxyde de carbone dans un fluide gazeux donné.

Ce procédé comprend les étapes suivantes :

- une étape de mélange de chlorure de palladium avec du potassium di-sulfite dans de l'eau distillée sous agitation,
- une étape de filtration de la suspension obtenue,

- une étape de lavage du précipité obtenu avec un solvant partiellement organique,
- une étape de séchage du précipité.

Le précipité résultant de la réaction est séché par exemple dans un dessiccateur pendant 12 heures à pression atmosphérique pour éliminer au maximum l'humidité.

A la suite de nombreux essais, les inventeurs ont fait varier les quantités respectives, en masse, de $PdCl_2$ et $K_2S_2O_5$ mises en présence pour préparer le complexe $K_2Pd(SO_3)_2$. Le rendement de la réaction, calculé par rapport à la masse initiale de $PdCl_2$ utilisée, peut varier entre 30 et 160 %. Cette valeur supérieure à 100 % est théoriquement possible car ce rendement a été calculé à partir de la masse initiale de $PdCl_2$, ceci en respectant la stoechiométrie connue de la réaction.

Les limites supérieures et inférieures des masses des deux réactifs à mettre en présence ont ainsi été précisées pour les valeurs respectives suivantes : masse de $PdCl_2$ comprise entre 1,05 g et 4,5 g ; masse de $K_2S_2O_5$ comprise entre 2,40 et 8,60 g.

On a obtenu de bons résultats avec une masse de $PdCl_2$ de 1,5 g et une masse de $K_2S_2O_5$ de 5 g dans 20 ml d'eau.

Le rapport massique des quantités des deux produits, $PdCl_2$ et $K_2S_2O_5$, est ainsi égal à 3, 33.

Des tests ont montré que, quelle que soit la masse de réactifs utilisés pour fabriquer le complexe di-potassium pallado bisulfite, la réaction au contact de monoxyde de carbone est la même, et on obtient une coloration uniforme brun foncé avec un front d'avancement net et un contraste très net entre la phase initiale jaune et la phase finale brun foncé.

L'invention concerne également un dispositif pour mesurer le taux volumique de monoxyde de carbone tel que décrit plus haut, caractérisé par le fait qu'il comprend un élément réactif pulvérulent comprenant un réactif sélectif adsorbé sur un gel de silice, et ayant une réaction colorée avec ledit monoxyde de carbone présent dans ledit fluide, et des moyens pour contrôler le passage d'un volume donné, dans un temps donné, dudit fluide.

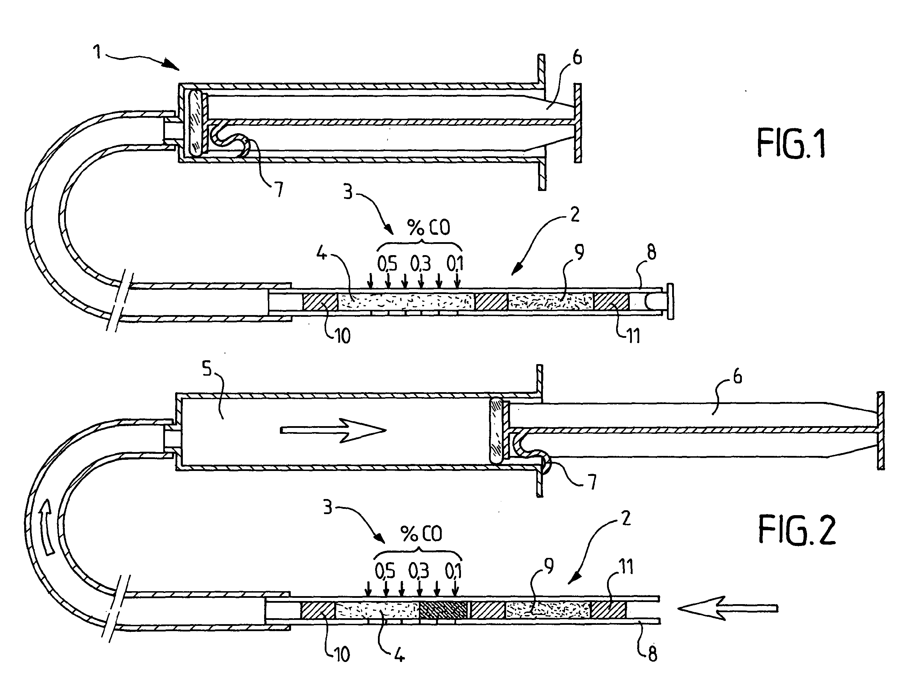

Selon un mode particulier non limitatif de la présente invention, ce dispositif comprend deux parties 1, 2, telles que montrées aux figures 1 et 2. Une partie constituée d'un système de prélèvement 1 et une partie constituée de l'élément réactif 2. Les deux parties forment un ensemble clos et étanche.

Tel qu'illustré aux figures 1 et 2, le système de prélèvement 1 du dispositif comprend des moyens pour créer un transfert du fluide gazeux à l'intérieur dudit dispositif, et des moyens pour réguler le débit dudit fluide contenant ledit monoxyde de carbone.

Encore et selon la présente invention et tel qu'illustré aux figures 1 et 2, l'élément réactif 2 se présente sous la forme d'un tube 8 transparent, par exemple de verre, contenant ledit réactif sélectif adsorbé sur un gel de silice 4, portant une échelle adaptée 3 par exemple imprimée sur le tube contenant la phase réactive, afin de mesurer la progression spatio-temporelle de la réaction entre ledit réactif et ledit monoxyde de carbone, de façon visuelle, et donc de lire directement sur l'échelle le pourcentage de CO en volume contenu dans le gaz d'échappement testé.

Des études ont été menées sur de nombreux gels de silice différents : ce sont des solides poreux, de diverses origines commerciales. Des performances très variables ont été constatées selon les caractéristiques desdits gels, différents physiquement par plusieurs paramètres intrinsèques ou circonstanciels qui sont : spectre granulométrique, densité, procédés d'introduction, de tassement et de maintien de la phase réactive, surface spécifique, diamètre des pores, traitements thermiques ou physiques divers effectués par les fabricants. Ce sont des gels de granulométrie comprise entre 0,06 et 0,3 mm qui conviennent, mais ces caractéristiques ne sont pas exclusives.

On a obtenu de bons résultats notamment avec les gels de silice connus sous la marque ACROS, d'une granulométrie de 0,06-0.2 mm, et le gel de silice NORMASIL, de granulométrie 0.063-0.2 mm.

En outre, les moyens pour créer ladite dépression à l'intérieur dudit dispositif tel qu'illustré aux figures 1 et 2, venant ainsi aspirer ledit fluide, comprennent une chambre de dépression 5, un piston étanche 6, et des moyens 7 pour bloquer le piston 6 une fois la dépression créée.

Encore selon la présente invention, les moyens, non limitatifs, pour réguler le débit dudit fluide contenant ledit monoxyde de carbone comprennent une phase tampon en amont de la phase réactive 4, constituée par exemple d'un gel de silice pur 9, ou d'une pièce cylindrique de polymère expansé, dont la nature et les dimensions sont choisies pour optimiser la perte de charge, qui rend voisines la vitesse de transfert physique du gaz d'échappement prélevé et la vitesse de la réaction chimique utilisée, et d'au moins deux filtres supports 10, 11, un de chaque côté de la phase réactive, par exemple en laine de verre fine pour maintenir l'ensemble en place.

La phase tampon, si constituée d'un gel de silice pur par exemple, a aussi pour but d'absorber l'humidité et ainsi d'éviter les interférences. On a obtenu de bons résultats avec une longueur de 1,5 cm pour la phase tampon de gel de silice pur.

Les filtres 10, 11, en laine de verre fine par exemple, ont essentiellement pour but de maintenir en place le réactif.

Afin d'éviter une quelconque dégradation du dispositif avant son utilisation, il est conservé en milieu inerte avant toute utilisation, et particulièrement l'élément réactif pulvérulent.

Selon la présente invention, le dispositif décrit plus haut fonctionne de la façon suivante :

- on rompt l'étanchéité du système afin de laisser pénétrer ledit fluide gazeux donné dans le dispositif,.
- on créée ladite dépression à l'intérieur dudit dispositif afin que ledit fluide gazeux passe dans ledit dispositif selon un volume donné et un temps donné.

La présente invention concerne également un élément à phase réactive pulvérulente comprenant entre 7 mg et 17 mg dudit complexe di-potassium pallado bisulfite proportionnellement adsorbé sur 0,5 et 3 g de gel de silice de granulométrie comprise entre 0.050 et 0.25 mm, et plus particulièrement entre 0,06 et 0,3 mm. L'élément à phase réactive pulvérulent comprend de 7 à 17 mg dudit complexe, selon le taux volumique de monoxyde de carbone supposé exister dans le fluide étudié. Mais ces valeurs ne sont pas limitatives.

La présente invention concerne plus particulièrement l'utilisation du dispositif décrit plus haut dans la détermination du taux de monoxyde de carbone dans les gaz d'échappement d'un système à combustion.

Naturellement, d'autres modes de mise en oeuvre, à la portée de l'homme de l'art, auraient pu être envisagés sans pour autant sortir du cadre de l'invention.

## Revendications

1. Procédé de mesure du taux volumique de monoxyde de carbone dans un fluide gazeux donné, **caractérisé par le fait qu'**on fait passer un volume donné dudit fluide, dans un temps donné, à travers un élément réactif (2) pulvérulent, comprenant un réactif sélectif adsorbé sur un gel de silice (4), et ayant une réaction colorée avec ledit monoxyde de carbone présent dans ledit fluide.

2. Procédé selon la revendication 1, **caractérisé par le fait que** le réactif sélectif (4) ayant une réaction colorée avec ledit monoxyde de carbone est le complexe di-potassium pallado bisulfite.

3. Procédé selon la revendication 1, **caractérisé par le fait que** le volume dudit fluide aspiré est de 5 à 35 ml et le temps de passage à travers l'élément réactif est de 2 mn à 10 mn.

4. Procédé selon la revendication 1, **caractérisé par le fait que** ledit gel de silice (4) présente une granulométrie comprise entre 0,06 et 0,3 mm

5. Procédé selon la revendication 1, **caractérisé par le fait que** l'élément réactif pulvérulent comprend une quantité de réactif sélectif entre 7 mg et 17 mg et une quantité de gel de silice entre 0,5 g et 3 g.

6. Procédé de préparation d'un réactif, conçu pour la mise en oeuvre du procédé selon la revendication 2, **caractérisé par le fait qu'**il comprend les étapes suivantes :

   - une étape de mélange de chlorure de palladium avec du potassium disulfite dans de l'eau distillée sous agitation,
   - une étape de filtration de la suspension obtenue,
   - une étape de lavage du précipité obtenu, avec un solvant partiellement organique,
   - une étape de séchage du précipité.

7. Dispositif (3) pour mesurer le taux volumique de monoxyde de carbone dans un fluide donné, conçu pour la mise en oeuvre du procédé selon la revendication 1, **caractérisé par le fait qu'**il comprend un élément réactif (4) pulvérulent, comprenant ledit réactif sélectif, adsorbé sur un gel de silice, et ayant une réaction colorée avec ledit monoxyde de carbone présent dans ledit fluide, et des moyens pour contrôler le passage d'un volume donné, dans un temps donné, dudit fluide.

8. Dispositif selon la revendication 7, **caractérisé par le fait qu'**il comprend deux parties, à savoir :

   - une partie constituée d'un système de prélèvement (1)
   - une partie constituée d'un élément réactif (2) pulvérulent, les deux parties formant un ensemble clos et étanche.

9. Dispositif selon la revendication 8, **caractérisé par le fait que** le système de prélèvement (1) comprend des moyens pour créer un transfert du fluide gazeux à l'intérieur dudit dispositif, et des moyens pour réguler le débit dudit fluide contenant ledit monoxyde de carbone.

10. Dispositif selon la revendication 8, **caractérisé par le fait que** l'élément réactif pulvérulent (2) se présente sous la forme d'un tube transparent (4), contenant ledit réactif, adsorbé sur un gel de silice, portant une échelle afin de mesurer la progression spatio temporelle de la réaction entre ledit réactif et ledit monoxyde de carbone.

**11.** Dispositif selon la revendication 9, **caractérisé par le fait que** les moyens pour créer ladite dépression à l'intérieur dudit dispositif et ainsi aspirer ledit fluide comprennent une chambre de dépression (5), un piston étanche (6), et des moyens (7) pour bloquer le piston une fois la dépression créée.

**12.** Dispositif selon la revendication 9, **caractérisé par le fait que** les moyens pour réguler le débit dudit fluide contenant ledit monoxyde de carbone comprennent une phase tampon en amont (9) de la phase réactive, et deux filtres supports(10, 11), un de chaque côté de la phase réactive.

**13.** Dispositif selon la revendication 8, **caractérisé par le fait qu'**au moins l'élément réactif pulvérulent est conservé en milieu inerte avant son utilisation.

**14.** Procédé de mesure selon la revendication 1, mise en oeuvre dans un dispositif, selon la revendication 9, **caractérisé par le fait que** :

- on rompt l'étanchéité du système afin de laisser pénétrer ledit fluide gazeux donné dans le dispositif,
- on crée ladite dépression à l'intérieur dudit dispositif, afin que ledit fluide gazeux passe dans ledit dispositif selon un volume donné et un temps donné.

**15.** Elément réactif pulvérulent selon la revendication 8, **caractérisé par le fait qu'**il comprend :

- 7 mg à 17 mg de complexe di-potassium pallado bisulfite,
- 0,5 g à 3 g de gel de silice de granulométrie 0.060 - 0.300 mm.

**16.** Utilisation d'un dispositif selon la revendication 7, dans la détermination du taux volumique de monoxyde de carbone dans les gaz d'échappement d'un système à combustion.

FIG.1

FIG.2

EP 1 710 573 A1

Office européen
des brevets

**RAPPORT DE RECHERCHE EUROPEENNE**

Numéro de la demande

EP 06 37 0011

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2 569 895 A (MAIN-SMITH JOHN DAVID ET AL) 2 octobre 1951 (1951-10-02) * le document en entier * | 1-15 | INV. G01N31/22 G01N33/00 |
| Y | US 5 733 505 A (GOLDSTEIN ET AL) 31 mars 1998 (1998-03-31) * abrégé * * colonne 3, ligne 40-50 * * colonne 5, ligne 55-65 * * colonne 6, ligne 61-65 * * colonne 7 * * revendications 1-6 * | 1-15 | |
| Y | US 4 043 934 A (SHULER ET AL) 23 août 1977 (1977-08-23) * colonne 2, ligne 30 - colonne 3, ligne 27 * * exemple 1 * | 1-15 | |
| Y | US 4 482 635 A (HERSKOVITZ ET AL) 13 novembre 1984 (1984-11-13) * colonnes 2-3 * | 1,6,7, 14,15 | **DOMAINES TECHNIQUES RECHERCHES (IPC)** G01N |
| Y | US 3 350 175 A (MCCONNAUGHEY PAUL WILLIS ET AL) 31 octobre 1967 (1967-10-31) * colonne 2, ligne 61 - colonne 3, ligne 4 * * colonne 3, ligne 60 - colonne 4, ligne 30; tableau 1 * | 1,6,7, 14,15 | |
| A | GB 585 322 A (JOHN DAVID MAIN-SMITH; GEORGE ALAN EARWICKER; SIDNEY ARTHUR DODD; GEOR) 4 février 1947 (1947-02-04) * le document en entier * | 1-15 | |

-/--

Le présent rapport a été établi pour toutes les revendications

| Lieu de la recherche | Date d'achèvement de la recherche | Examinateur |
|---|---|---|
| Munich | 2 août 2006 | Michalitsch, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................
& : membre de la même famille, document correspondant

EPO FORM 1503 03.82 (P04C02)

**Office européen des brevets**

# RAPPORT DE RECHERCHE EUROPEENNE

Numéro de la demande

EP 06 37 0011

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | GB 656 257 A (GEORGE MARTIN SHEPHERD) 15 août 1951 (1951-08-15) <br> * page 1 * <br> * page 6, colonne de droite - page 8, colonne de gauche * <br> * revendications 1-4 * <br> * figure 2 * <br> ----- | 1-15 | |

**DOMAINES TECHNIQUES RECHERCHES (IPC)**

Le présent rapport a été établi pour toutes les revendications

| Lieu de la recherche | Date d'achèvement de la recherche | Examinateur |
|---|---|---|
| Munich | 2 août 2006 | Michalitsch, R |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE**
**RELATIF A LA DEMANDE DE BREVET EUROPEEN NO.**     EP 06 37 0011

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-08-2006

| Document brevet cité au rapport de recherche | | Date de publication | Membre(s) de la famille de brevet(s) | | Date de publication |
|---|---|---|---|---|---|
| US 2569895 | A | 02-10-1951 | AUCUN | | |
| US 5733505 | A | 31-03-1998 | AUCUN | | |
| US 4043934 | A | 23-08-1977 | AUCUN | | |
| US 4482635 | A | 13-11-1984 | AUCUN | | |
| US 3350175 | A | 31-10-1967 | DE | 1239872 B | 03-05-1967 |
| | | | GB | 1057984 A | 08-02-1967 |
| GB 585322 | A | 04-02-1947 | AUCUN | | |
| GB 656257 | A | 15-08-1951 | AUCUN | | |

EPO FORM P0460

Pour tout renseignement concernant cette annexe : voir Journal Officiel de l'Office européen des brevets, No.12/82